# EUROPÄISCHE PATENTSCHRIFT

(11) **EP 2 219 778 B1**
(45) Veröffentlichungstag und Bekanntmachung des Hinweises auf die Patenterteilung: **13.07.2011**
(21) Anmeldenummer: 08859853.7
(22) Anmeldetag: 14.10.2008
(51) Int. Cl.: B01J 19/12

(54) **VERFAHREN ZUR DURCHFÜHRUNG CHEMISCHER REAKTIONEN MIT HILFE EINES INDUKTIV ERWÄRMTEN HEIZMEDIUMS**
METHOD FOR CARRYING OUT CHEMICAL REACTIONS WITH THE AID OF AN INDUCTIVELY HEATED HEATING MEDIUM
PROCÉDÉ POUR EFFECTUER DES RÉACTIONS CHIMIQUES À L'AIDE D'UN MILIEU CHAUFFANT ÉCHAUFFÉ PAR INDUCTION

(30) Priorität: 11.12.2007 DE 102007059967
(43) Veröffentlichungstag der Anmeldung: 25.08.2010
(73) Patentinhaber: Henkel AG & Co. KGaA, 40589 Düsseldorf (DE)
(72) Erfinder: FRIESE, Carsten, 40219 Düsseldorf (DE); KIRSCHNING, Andreas, 29221 Celle (DE); WICHELHAUS, Jürgen, 42113 Wuppertal (DE); CEYLAN, Sascha Volhan, 30169 Hannover (DE)
(86) Internationale Anmeldenummer: PCT/EP2008/063763
(87) Internationale Veröffentlichungsnummer: WO 2009/074373

(56) Entgegenhaltungen:
- WO-A-95/21126
- WO-A-03/042315
- DE-A1-102005 051 637

## Beschreibung

Die vorliegende Erfindung liegt auf dem Gebiet der chemischen Synthese und betrifft ein Verfahren zum Durchführen einer chemischen Reaktion mit Hilfe eines induktiv erwärmten Heizmediums.

Zur Durchführung thermisch induzierbarer chemischer Reaktionen sind unterschiedliche Wege bekannt, um die Reaktanten zu erwärmen. Am weitesten verbreitet ist ein Erwärmen durch Wärmeleitung. Dabei befinden sich die Reaktanten in einem Reaktor, wobei entweder die Wände des Reaktors selbst erwärmt werden oder wobei in dem Reaktor Wärme übertragende Elemente wie beispielsweise Heizschlangen oder Wärmetauscherrohre bzw. -platten eingebaut sind. Dieses Verfahren ist vergleichsweise träge, so dass zum einen das Aufheizen der Reaktanten langsam erfolgt und zum anderen die Wärmezufuhr nicht schnell unterbunden bzw. sogar durch eine Kühlung ersetzt werden kann. Eine Alternative hierzu besteht darin, die Reaktanten durch Einstrahlen von Mikrowellen in die Reaktanten selbst oder in ein Medium, das die Reaktanten enthält, zu erwärmen. Mikrowellengeneratoren stellen jedoch ein erhebliches Sicherheitsrisiko dar, da sie apparativ aufwendig sind und die Gefahr des Austritts von Strahlung besteht.

Im Gegensatz hierzu stellt die vorliegende Erfindung ein Verfahren zur Verfügung, bei dem man ein Reaktionsmedium dadurch erwärmt, dass man es in Kontakt mit einem durch elektromagnetische Induktion erwärmbaren Heizmedium bringt, das man "von außen" durch elektromagnetische Induktion mit Hilfe eines Induktors erwärmt.

Das Verfahren des induktiven Erhitzens wird schon länger in der Industrie verwendet. Die häufigsten Anwendungen sind Schmelzen, Härten, Sintern und die Wärmebehandlung von Legierungen. Aber auch Prozesse wie Kleben, Schrumpfen oder Verbinden von Bauteilen sind bekannte Anwendungen dieser Heiztechnik.

Aus der deutschen Patentanmeldung DE 198 00 294 sind Verfahren zur Isolierung und zur Analyse von Biomolekülen bekannt, wobei diese Biomoleküle an die Oberfläche von induktiv aufheizbaren Magnetpartikeln gebunden werden. Dieses Dokument führt aus:

"Das Wirkprinzip besteht darin, auf die Oberfläche einer funktionellen Polymermatrix, in die die induktiv aufheizbaren magnetischen Kolloide bzw. feindispersen Magnetteilchen eingekapselt sind, Biomoleküle adsorptiv oder covalent zu binden, die in der Lage sind, Analyten wie z.B. DNA/RNA-Sequenzen, Antikörper, Antigene, Proteine, Zellen, Bakterien, Viren oder Pilzsporen gemäß dem komplementären Affinitätsprinzip zu binden. Nach der Bindung der Analyten auf der Matrix können die Magnetpartikel in einem hochfrequenten magnetischen Wechselfeld auf die für die Analytik, Diagnostik und Therapie relevanten Temperaturen von vorzugsweise 40 bis 120 °C aufgeheizt werden." Im weiteren geht dieses Dokument auf die technische Auslegung von Spulensystemen und Hochfrequenzgeneratoren ein, die in diesem Verfahren verwendet werden können. Das genannte Dokument beschreibt also den Einsatz induktiv erwärmbarer Partikel bei der Analyse komplexer biologischer Systeme oder Biomoleküle.

DE 10 2005 051637 beschreibt ein Reaktorsystem mit einem mikrostrukturierten Reaktor sowie Verfahren zur Durchführung einer chemischen Reaktion in einem solchen Reaktor. Dabei wird der Reaktor als solcher durch elektromagnetische Induktion aufgeheizt. Der Wärmeübergang in das Reaktionsmedium erfolgt über die aufgeheizten Reaktorwände. Dies begrenzt zum einen die Größe der Fläche, die zum Erwärmen des Reaktionsmediums zur Verfügung steht. Zum anderen ist es erforderlich, Teile des Reaktors mit zu erwärmen, die nicht in direktem Kontakt mit dem Reaktionsmedium stehen.

US 5,110,996 beschreibt die Herstellung von Vinylidenfluorid durch Umsetzung von Dichlordifluormethan mit Methan in der Gasphase in einem erhitzten Reaktor. Der Reaktor ist mit einem nicht-metallischen Füllmaterial gefüllt. Der Reaktionsraum, der dieses Füllmaterial enthält, ist von einer metallischen Hülle umgeben, die von außen durch elektromagnetische Induktion erhitzt wird. Der Reaktionsraum selbst wird also von außen beheizt, wodurch sich das Füllmaterial durch Wärmestrahlung und/oder Wärmeleitung mit der Zeit ebenfalls erhitzt. Eine unmittelbare Erwärmung des von den Reaktanten umströmten Füllmaterials durch elektromagnetische Induktion erfolgt selbst dann nicht, wenn dieses Füllmaterial elektrisch leitend ist, da die metallische Reaktorwand die elektromagnetischen Felder der Induktionsspule abschirmt.

WO 95/21126 offenbart ein Verfahren für die Herstellung von Cyanwasserstoff in der Gasphase aus Ammoniak und einem Kohlenwasserstoff mit Hilfe eines metallischen Katalysators. Der Katalysator befindet sich innerhalb des Reaktionsraums, so dass er vor den Reaktanten umströmt wird. Er wird von außen durch elektromagnetische Induktion mit einer Frequenz von 0,5 bis 30 MHz, also mit einem Wechselfeld im Hochfrequenzbereich, erwärmt. Dabei zitiert dieses Dokument das vorstehend genannte Dokument US 5,110,996 mit der Bemerkung, dass üblicherweise induktives Heizen im Frequenzbereich von etwa 0,1 bis 0,2 MHz durchgeführt wird. Diese Angabe ist jedoch in dem zitierten US 5,110,996 nicht enthalten, so dass unklar ist, worauf sie sich bezieht.

WO 00/38831 befasst sich mit gesteuerten Adsorptions- und Desorptionsprozessen, wobei die Temperatur des Adsorbermaterials durch elektromagnetische Induktion gesteuert wird,

Gegenstand der vorliegenden Erfindung ist ein Verfahren zum Durchführen einer chemischen Reaktion zur Herstellung einer Zielverbindung durch Erwärmen eines Reaktionsmediums enthaltend mindestens einen ersten Reaktanten in einem Reaktor, wodurch eine chemische Bindung innerhalb des ersten Reaktanten oder zwischen dem ersten und einem zweiten Reaktanten gebildet oder verändert wird, wobei man das Reaktionsmedium in Kontakt mit einem durch elektromagnetische Induktion erwärmbaren festen Heizmedium bringt, das sich innerhalb des Reaktors befindet und das von dem Reaktionsmedium umgeben ist, und das Heizmedium durch elektromagnetische Induktion mit Hilfe eines Induktors erwärmt, wobei sich aus dem ersten Reaktanten oder aus dem ersten und einem zweiten Reaktanten die Zielverbindung bildet und wobei man die Zielverbindung von dem Heizmedium abtrennt, wobei das Reaktionsmedium im Reaktor als Flüssigkeit vorliegt und der Induktor ein Wechselfeld mit einer Frequenz im Bereich von 1 bis 100 khz erzeugt.

Die chemische Reaktion findet also durch Erwärmen eines Reaktionsmediums statt, das mindestens einen ersten Reaktanten enthält. Dies schließt die Möglichkeit mit ein, dass das Reaktionsmedium, beispielsweise eine Flüssigkeit, selbst an der Reaktion beteiligt ist und damit einen Reaktanten darstellt. Das gesamte Reaktionsmedium kann also aus einem Reaktanten bestehen. Weiterhin kann ein Reaktant in dem Reaktionsmedium gelöst oder dispergiert sein, wobei das Reaktionsmedium selbst inert sein oder seinerseits einen Reaktanten darstellen kann. Oder ein, zwei oder mehr Reaktanten liegen gelöst oder dispergiert in einem Reaktionsmedium vor, das selbst nicht durch die chemische Reaktion verändert wird.

Dabei kann das Reaktionsmedium aus einem einzigen Reaktanten bestehen oder diesen enthalten, wobei Moleküle des Reaktanten miteinander reagieren oder wobei eine Veränderung des chemischen Bindungssystems in den einzelnen Molekülen des Reaktanten selbst stattfinden kann. In beiden Fällen wird der Reaktant chemisch verändert. Im allgemeinen Fall treten jedoch zwei oder mehr Reaktanten miteinander in Reaktion, wobei chemische Bindungen innerhalb und/oder zwischen den einzelnen Reaktanten umgelagert oder gebildet werden.

Das feste Heizmedium ist von dem Reaktionsmedium umgeben. Dies kann bedeutet, dass sich das feste Heizmedium, von möglichen Randzonen abgesehen, innerhalb des Reaktionsmediums befindet, z.B. wenn das Heizmedium in Form von Partikeln, Spänen, Drähten, Netzen, Wolle, Füllkörpern etc. vorliegt. Dies kann aber auch bedeuten, dass das Reaktionsmedium das Heizmedium durch eine Vielzahl von Hohlräumen im Heizmedium durchströmt, wenn dieses beispielsweise aus einer oder mehreren Membranen, einem Bündel von Rohren, einer aufgerollten Metallfolie, Fritten, porösen Füllkörpern oder aus einem Schaum besteht. Auch hierbei ist das Heizmedium im wesentlichen von dem Reaktionsmedium umgeben, da der größte Teil seiner Oberfläche (90 % oder mehr) im Kontakt mit dem Reaktionsmedium steht. Im Gegensatz hierzu ist bei einem Reaktor, dessen Außenwand durch elektromagnetische Induktion erwärmt wird (wie beispielsweise gemäß dem zitierten Dokument US 5110996), nur die innere Reaktoroberfläche mit dem Reaktionsmedium in Kontakt.

Die Wand des Reaktors ist aus einem Material, das das die vom Induktor erzeugte elektromagnetische Wechselfeld nicht abschirmt bzw. absorbiert und daher nicht selbst erwärmt wird. Metalle sind also ungeeignet. Beispielsweise kann es aus Kunststoff, Glas oder Keramik (wie beispielsweise Siliciumcarbid oder Siliciumnitrid) bestehen. Letztere ist insbesondere für Reaktionen bei hoher Temperatur (500 - 600 °C) und/oder unter Druck geeignet.

Die vorstehend beschriebene Verfahrensweise hat den Vorteil, dass die thermische Energie zur Durchführung der chemischen Reaktion nicht durch Oberflächen wie beispielsweise die Reaktorwände, Heizschlangen, Wärmetauscherplatten oder ähnliches in das Reaktionsmedium eingebracht wird, sondern direkt im Volumen des Reaktors erzeugt wird. Das Verhältnis von erwärmter Oberfläche zu Volumen des Reaktionsmediums kann dabei wesentlich größer werden als bei einer Heizung über Wärme übertragende Oberflächen, wie es beispielsweise auch gemäß der eingangs zitierten DE 10 2005 051637 der Fall ist. Zusätzlich wird der Wirkungsgrad von elektrischem Strom zu Heizleistung verbessert. Durch Einschalten des Induktors kann die Wärme in dem gesamten festen Heizmedium, das über eine sehr große Oberfläche mit dem Reaktionsmedium in Kontakt steht, erzeugt werden. Bei Abschalten des Induktors wird der weitere Wärmeeintrag sehr rasch unterbunden. Dies erlaubt eine sehr gezielte Reaktionsführung.

Nach Bildung der Zielverbindung wird diese von dem Heizmedium abgetrennt. Im besten Fall wird dabei die Zielverbindung in reiner Form, also ohne Lösungsmittel und mit nicht mehr als den üblichen Verunreinigungen isoliert. Die Zielverbindung kann aber auch im Gemisch mit Reaktanten oder als Lösung im Reaktionsmedium vom Heizmedium abgetrennt und erst danach durch weitere Aufarbeitung isoliert oder in ein anderes Lösungsmittel überführt werden, sofern dies gewünscht wird. Das Verfahren dient also der präparativen Herstellung der Zielverbindung, um diese weiter verwenden zu können.

Im Gegensatz hierzu stehen Verfahren, bei denen zwar durch elektromagnetische Induktion eines Heizmediums ebenfalls eine chemische Reaktion gestartet wird, diese Reaktion jedoch nicht der Herstellung einer Zielverbindung dient, die nach Reaktionsende von dem Heizmedium abgetrennt wird. Ein Beispiel hierfür ist das Härten von Harzsystemen, wobei die Härtungsreaktion an Partikeln gestartet wird, die in dem Harzsystem dispergiert sind und die durch elektromagnetische Induktion erhitzt werden. Hierbei verbleiben diese Partikel im ausgehärteten Harzsystem und es wird keine definierte Zielverbindung isoliert. Dasselbe gilt für den umgekehrten Fall, dass eine Klebeverbindung durch das induktive Erhitzen von Partikeln wieder gelöst wird, die sich in der Klebstoffmatrix befinden. Hierbei kann zwar eine chemische Reaktion stattfinden, es werden jedoch keine Zielverbindungen isoliert.

Das Heizmedium besteht aus einem elektrisch leitfähigen Material, das sich bei Einwirken eines elektromagnetischen Wechselfelds erwärmt. Es ist vorzugsweise ausgewählt aus Materialien, die im Vergleich zu ihrem Volumen eine sehr große Oberfläche aufweisen. Beispielsweise kann das Heizmedium ausgewählt sein aus jeweils elektrisch leitfähigen Spänen, Drähten, Netzen, Wolle, Membranen, porösen Fritten, Rohrbündeln (aus drei oder mehr Rohren), aufgerollter Metallfolie, Schäumen, Füllkörper wie beispielsweise Granulat oder Kugeln, Raschig-Ringe und insbesondere aus Partikeln, die vorzugsweise einen mittleren Durchmesser von nicht mehr als 1 mm aufweisen. Beispielsweise können als Heizmedium metallische Mischelemente eingesetzt werden, wie sie für Statikmischer verwendet werden. Um durch elektromagnetische Induktion erwärmbar zu sein, ist das Heizmedium elektrisch leitfähig, beispielsweise metallisch (wobei es diamagnetisch sein kann,) oder es zeigt eine gegenüber Diamagnetismus verstärkte Wechselwirkung mit einem Magnetfeld und ist insbesondere ferromagnetisch, ferrimagnetisch, paramagnetisch oder superparamagnetisch. Dabei ist es unerheblich, ob das Heizmedium organischer oder anorganischer Natur ist oder ob es sowohl anorganische als auch organische Komponenten enthält.

In einer bevorzugten Ausführungsform ist das Heizmedium ausgewählt aus Partikeln elektrisch leitfähiger und/oder magnetisierbarer Festkörper, wobei die Partikel eine mittlere Teilchengröße im Bereich von 1 bis 1000, insbesondere von 10 bis 500 nm haben. Die mittlere Teilchengröße und bei Bedarf auch die Teilchengrößenverteilung ist beispielsweise durch Lichtstreuung bestimmbar. Vorzugsweise wählt man magnetische Partikel, beispielsweise ferromagnetische oder superparamagnetische Partikel, die eine möglichst geringe Remanenz bzw. Restmagnetisierung aufweisen. Dies hat den Vorteil, dass die Partikel nicht aneinander haften. Die magnetischen Partikel können beispielsweise in Form sogenannter "Ferrofluide" vorliegen, also Flüssigkeiten, in denen ferromagnetische Partikel im nano-Größenmaßstab dispergiert sind. Die flüssige Phase des Ferrofluids kann dann als Reaktionsmedium dienen.

Magnetisierbare Partikel, insbesondere ferromagnetische Partikel, welche die gewünschten Eigenschaften aufweisen, sind im Stand der Technik bekannt und kommerziell erhältlich. Beispielsweise seien die kommerziell erhältlichen Ferrofluide genannt. Beispiele für die Herstellung magnetischer nano-Partikel, die im Rahmen des erfindungsgemäßen Verfahrens verwendet werden können, können dem Aufsatz von Lu, Salabas und Schüth: "Magnetische nano-Partikel: Synthese, Stabilisierung, Funktionalisierung und Anwendung", Angew. Chem. 2007, 119, Seiten 1242 bis 1266 entnommen werden.

Geeignete magnetische nano-Partikel sind mit unterschiedlichen Zusammensetzungen und Phasen bekannt. Beispielsweise seien genannt: reine Metalle wie Fe, Co und Ni, Oxide wie Fe₃O₄ und gamma-Fe₂O₃, spinellartige Ferromagnete wie MgFe₂O₄ MnFe₂O₄ und CoFe₂O₄ sowie Legierungen wie C_{O}Pt₃ und FePt. Die magnetischen nano-Partikel können homogen aufgebaut sein oder eine Kern-Schale-Struktur besitzen. In letzterem Fall können Kern und Schale aus unterschiedlichen ferromagnetischen oder auch antiferromagnetischen Materialien bestehen. Es sind jedoch auch Ausführungsformen möglich, bei denen mindestens ein magnetisierbarer Kern, der beispielsweise ferromagnetisch, antiferromagnetisch, paramagnetisch oder superparamagnetisch sein kann, von einem nicht magnetischen Material umgeben ist. Dieses Material kann beispielsweise ein organisches Polymer darstellen. Oder die Schale besteht aus einem anorganischen Material wie beispielsweise Kieselsäure bzw. SiO₂. Durch eine solche Beschichtung kann eine chemische Wechselwirkung des Reaktionsmediums bzw. der Reaktanten mit dem Material der magnetischen Partikel selbst verhindert werden. Weiterhin kann das Material der Schale oberflächlich funktionalisiert werden, ohne dass das Material des magnetisierbaren Kerns in Wechselwirkung mit der funktionalisierenden Spezies tritt. Dabei können auch mehrer Partikel des Kernmaterials gemeinsam in eine derartige Schale eingeschlossen sein.

Als Heizmedium können beispielsweise nanoskalige Teilchen aus superparamagnetischen Stoffen eingesetzt werden, die ausgewählt sind aus Aluminium, Cobalt, Eisen, Nickel oder deren Legierungen, Metalloxiden vom Typ des n-Maghemits (gamma-Fe₂O₃), n-Magnetits (Fe₃O₄) oder der Ferritte vom Typ des MeFe₂O₄, wobei Me ein zweiwertiges Metall ausgewählt aus Mangan, Kupfer, Zink, Cobalt, Nickel, Magnesium, Calcium oder Cadmium ist. Vorzugsweise haben diese Teilchen eine durchschnittliche Teilchengröße von ≤ 100 nm, vorzugsweise ≤ = 51 nm und insbesondere bevorzugt ≤ 30 nm.

Beispielsweise ist ein Material geeignet, das von der Firma Evonik (früher Degussa) unter der Bezeichnung MagSilica^{R} erhältlich ist. Bei diesem Material sind Eisenoxidkristalle mit einer Größe von 5 bis 30 nm in eine amorphe Kieselsäurematrix eingebettet. Besonders geeignet sind solche Eisenoxid-Siliciumdioxid-Kompositpartikel, die in der deutschen Patentanmeldung DE 101 40 089 näher beschrieben sind.

Diese Partikel können superparamagnetische Eisenoxid-Domänen mit einem Durchmesser von 3 bis 20 nm enthalten. Hierunter sind räumlich von einander getrennte superparamagnetische Bereiche zu verstehen. In diesen Domänen kann das Eisenoxid in einer einheitlichen Modifikation oder in verschiedenen Modifikationen vorliegen. Eine besonders bevorzugte superparamagnetische Eisenoxid-Domäne ist gamma-Fe₂O₃, Fe₃O₄ und Mischungen hiervon.

Der Anteil der superparamagnetischen Eisenoxid-Domänen dieser Partikel kann zwischen 1 und 99,6 Gew.-% liegen. Die einzelnen Domänen sind durch eine nichtmagnetisierbare Siliciumdioxid-Matrix voneinander getrennt und/oder von dieser umgeben. Bevorzugt ist der Bereich mit einem Anteil an superparamagnetischen Domänen > 30 Gew.-%, besonders bevorzugt > 50 Gew.-%. Mit dem Anteil der superparamagnetischen Bereiche nimmt auch die erzielbare magnetische Wirkung der erfindungsgemäßen Partikel zu. Neben der räumlichen Trennung der superparamagnetischen Eisenoxid-Domänen kommt der Siliciumdioxid-Matrix auch die Aufgabe zu, die Oxidationsstufe der Domäne zu stabilisieren. So wird zum Beispiel Magnetit als superparamagnetische Eisenoxidphase durch eine Siliciumdioxidmatrix stabilisiert. Diese und weitere Eigenschaften dieser für die vorliegende Erfindung besonders geeigneten Partikel sind in DE 101 40 089 und in WO 03/042315 näher ausgeführt.

Weiterhin sind als Heizmedium nanoskalige Ferrite einsetzbar, wie sie beispielsweise aus der WO 03/054102 bekannt sind. Diese Ferrite weisen eine Zusammensetzung (M^{a}_{1-x-y} M^{b}ₓ Fe^{II}_{y}) Fe^{III}₂O₄ auf, bei der
M^{a} ausgewählt ist aus Mn, Co, Ni, Mg, Ca, Cu, Zn, Y und V,
M^{b} ausgewählt ist aus Zn und Cd,
x für 0,05 bis 0,95, bevorzugt 0,01 bis 0,8 steht,
y für 0 bis 0,95 steht und
die Summe aus x und y höchstens 1 beträgt.

Die durch elektromagnetische Induktion erwärmbaren Partikel können ohne weitere Zusatzstoffe das Heizmedium darstellen. Es ist jedoch auch möglich, die durch elektromagnetische Induktion erwärmbaren Partikel mit anderen Partikeln zu mischen, die nicht durch elektromagnetische Induktion erwärmbar sind. Beispielsweise kann es sich hierbei um Sand handeln. Die induktiv erwärmbaren Partikel können also durch nicht induktiv erwärmbare Partikel verdünnt werden. Hierdurch kann eine verbesserte Temperaturkontrolle erreicht werden. In einer weiteren Ausführungsform können die induktiv erwärmbaren Partikel in Abmischungen mit nicht-induktiv erwärmbaren Partikeln vorliegen, die für die durchzuführende chemische Reaktion katalytische Eigenschaften haben oder sich in sonstiger Weise an der chemischen Reaktion beteiligen. Diese Partikel werden dann nicht unmittelbar durch elektromagnetische Induktion erwärmt, sondern mittelbar dadurch, dass sie durch den Kontakt mit den erwärmbaren Partikeln oder durch Wärmeübertragung durch das Reaktionsmedium erwärmt werden.

Werden nanoskalige durch elektromagnetische Induktion erwärmbare Partikel mit gröberen nicht induktiv erwärmbaren Partikeln vermischt, kann dies zu einer Verringerung der Packungsdichte des Heizmediums führen. Bei Ausführungsformen, bei denen das Reaktionsmedium eine Packung aus dem Heizmedium durchströmt, kann dies eine erwünschte Reduktion des Druckverlusts im durchströmten Reaktor zur Folge haben.

Das feste Heizmedium kann oberflächlich mit einer für die beabsichtigte chemische Reaktion katalytisch wirkenden Substanz belegt sein. Beispielsweise kann es sich hierbei um organische Moleküle oder um Biomoleküle mit Enzymwirkung handeln. Dann ist dafür Sorge zu tragen, dass durch die elektromagnetische Induktion das Heizmedium nicht so stark erwärmt wird, dass diese Moleküle ihre Enzymwirkung verlieren.

Insbesondere kann das induktiv erwärmtbare Heizmedium mit Metallatomen bzw. Metallverbindungen belegt sein, deren katalytische Wirkung bekannt ist. Beispielsweise können dies Atome oder Verbindungen von Metallen der Lanthanidenreihe, insbesondere Sm oder Ce, Fe, Co, Ni oder Edelmetalle, vorzugsweise Platinmetalle und insbesondere Pd oder Pt sein.

Zum Belegen mit katalytisch wirksamen Atomen oder Verbindungen sind insbesondere Partikel geeignet, die magnetisierbare Domänen in einer Siliciumdioxid- bzw. Kieselsäurematrix enthalten, beispielsweise die weiter oben beschriebenen Kompositpartikel aus Eisenoxid und Siliciumdioxid. Die Siliciumdioxid-Hülle trägt, wie in WO 03/042315 näher beschrieben, reaktive OH-Gruppen, deren Reaktivität zum Fixieren der katalytisch wirksamen Substanz auf der Partikeloberfläche ausgenutzt werden kann. Einige Beispiele hierfür werden im experimentellen Teil angegeben.

Prinzipiell kann die chemische Reaktion kontinuierlich oder chargenweise durchgeführt werden. Führt man die Reaktion chargenweise durch, geht man vorzugsweise so vor, dass man während der Reaktion das Reaktionsmedium und das induktiv erwärmte feste Heizmedium relativ zu einander bewegt. Beim Verwenden eines partikelförmigen Heizmediums kann dies insbesondere dadurch erfolgen, dass man das Reaktionsmedium zusammen mit dem Heizmedium rüht oder das Heizmedium in dem Reaktionsmedium verwirbelt. Verwendet man beispielsweise Netze oder Wolle aus einem fadenförmig ausgestalteten Heizmedium, kann beispielsweise der Reaktionsbehälter, der das Reaktionsmedium und das Heizmedium enthält, geschüttelt werden.

Eine bevorzugte Ausführungsform einer chargenweise durchgeführten Reaktion besteht darin, dass sich das Reaktionsmedium zusammen mit Partikeln des Heizmediums in einem Reaktionsbehälter befindet und mit Hilfe eines im Reaktionsmedium befindlichen Bewegungselements bewegt wird, wobei das Bewegungselement als Induktor eingerichtet ist, durch den die Partikel des Heizmediums durch elektromagnetische Induktion erwärmt werden. In dieser Ausführungsform befindet sich also der Induktor innerhalb des Reaktionsmediums. Das Bewegungselement kann beispielsweise als Rührer oder als sich auf und ab bewegender Stempel ausgebildet sein.

Man kann zusätzlich vorsehen, dass der Reaktor während der chemischen Reaktion von außen gekühlt wird. Dies ist insbesondere bei Chargenbetrieb möglich, wenn, wie vorstehend angegeben, der Induktor in das Reaktionsmedium eintaucht. Das Einspeisen des elektromagnetischen Wechselfelds in den Reaktor wird dann nicht durch die Kühleinrichtung behindert.

Eine Kühlung des Reaktors kann von innen über Kühlschlangen oder Wärmetauscher oder vorzugsweise von außen erfolgen. Zur Kühlung kann man beispielsweise ggf. vorgekühltes Wasser oder auch eine Kühlmischung einsetzen, deren Temperatur unterhalb von 0 °C liegt. Beispiele solcher Kühlmischungen sind Eis-Kochsalz-Gemische, Methanol / Trockeneis oder flüssiger Stickstoff. Durch die Kühlung lässt sich ein Temperaturgradient zwischen der Reaktorwand und dem induktiv erwärmten Heizmedium herstellen. Dieser ist besonders ausgeprägt, wenn man eine Kühlmischung mit einer Temperatur deutlich unterhalb von 0 °C einsetzt, beispielsweise Methanol / Trockeneis oder flüssigen Stickstoff. Das Reaktionsmedium, das durch das induktiv erwärmte Heizmedium aufgewärmt wird, wird dann wieder extern abgekühlt. Die chemische Reaktion des Reaktanten findet dann immer nur dann statt, wenn er Kontakt mit dem Heizmedium hat oder sich zumindest in dessen unmittelbarer Nähe befindet. Bei der Reaktion entstandene Produktspezies gelangen durch die Relativbewegung des Reaktionsmediums zum Heizmedium rasch in kühlere Bereiche des Reaktionsmediums, so dass ihre thermische Weiterreaktion gehemmt ist. Auf diese Weise kann man bei mehreren möglichen Reaktionswegen des oder der Reaktanten einen erwünschten Reaktionsweg kinetisch selektieren.

In einer alternativen Ausführungsform führt man die chemische Reaktion kontinuierlich in einem Durchflussreaktor durch, der zumindest teilweise mit dem festen Heizmedium gefüllt ist und hierdurch mindestens eine durch elektromagnetische Induktion erwärmbare Heizzone aufweist, wobei das Reaktionsmedium den Durchflussreaktor kontinuierlich durchströmt und wobei sich der Induktor außerhalb des Reaktors befindet. Hierbei umströmt das Reaktionsmedium das Heizmedium, z.B. wenn dieses in Form von Partikeln, Spänen, Drähten, Netzen, Wolle, Füllkörpern etc. vorliegt. Oder das Reaktionsmedium durchströmt das Heizmedium durch eine Vielzahl von Hohlräumen im Heizmedium, wenn dieses beispielsweise aus einer oder mehreren Membranen, Fritten, porösen Füllkörpern oder aus einem Schaum besteht.

Vorzugsweise ist der Durchflussreaktor als Rohrreaktor ausgeführt. In diesem Fall kann der Induktor den Reaktor vollständig oder zumindest teilweise umgeben. Das vom Induktor erzeugte elektromagnetische Wechselfeld wird dann allseitig oder zumindest von mehreren Stellen aus in den Reaktor eingeleitet.

Unter "kontinuierlich" wird hierbei wie üblich eine Reaktionsführung verstanden, bei der das Reaktionsmedium den Reaktor zumindest über einen solchen Zeitraum durchströmt, dass ein Gesamtvolumen an Reaktionsmedium, das groß ist im Vergleich zum inneren Volumen des Reaktors selbst, den Reaktor durchströmt hat, bevor man den Fluss des Reaktionsmediums unterbricht. "Groß" in diesem Sinne bedeutet: "mindestens doppelt so groß". Selbstverständlich hat auch eine solche kontinuierlich durchgeführte Reaktion ein Beginn und ein Ende.

Bei dieser kontinuierlichen Verfahrensweise in einem Durchflussreaktor ist es möglich, dass der Reaktor mehrere Heizzonen aufweist. Beispielsweise können unterschiedliche Heizzonen unterschiedlich stark erwärmt werden. Dies kann entweder durch die Anordnung unterschiedlicher Heizmedien in dem Durchflussreaktor oder durch unterschiedlich ausgelegte Induktoren entlang des Reaktors erfolgen.

Bei der Verwendung mindestens zweier Heizzonen besteht eine besondere Ausführungsform darin, dass der Durchflussreaktor eine erste und eine zweite Heizzone aufweist, wobei die in Strömungsrichtung des Reaktionsmediums erste Heizzone ein nicht mit einer katalytisch wirkenden Substanz belegtes Heizmedium enthält, während die in Strömungsrichtung des Reaktionsmediums zweite Heizzone ein mit einer katalytisch wirkenden Substanz belegtes Heizmedium enthält. In einer alternativen Ausführungsform wählt man die umgekehrte Anordnung für das katalytisch wirksame und das nicht katalytisch wirksame Heizmedium. Hierdurch ist es möglich, vor oder nach einem katalytisch angeregten Reaktionsschritt einen weiteren, nicht katalytisch angeregten Reaktionsschritt vorzunehmen.

Man kann auch so verfahren, dass man das Lösungsmittel oder das Reaktionsmedium zunächst konventionell vorerwärmt, bevor es zur Durchführung der Reaktion mit dem Heizmedium in Kontakt kommt.

Falls gewünscht, kann nach der (letzten) Heizzone eine Kühlzone vorgesehen werden, beispielsweise in Form eines Kühlmantels um den Reaktor.

Weiterhin kann vorgesehen werden, dass das Reaktionsmedium nach Verlassen der Heizzone mit einer Absorbersubstanz in Kontakt gebracht wird, die Nebenprodukte oder Verunreinigungen aus dem Reaktionsmedium entfernt. Beispielsweise kann es sich hierbei um ein Molekularsieb handeln, das von dem Reaktionsmedium nach Verlassen der Heizzone durchströmt wird. Hierdurch ist eine Produktreinigung unmittelbar nach dessen Herstellung möglich.

Je nach Geschwindigkeit der chemischen Reaktion kann man die Produktausbeute ggf. dadurch erhöhen, dass das Reaktionsmedium nach Durchströmen des festen Heizmediums zumindest teilweise zum erneuten Durchströmen des festen Heizmediums zurückgeführt wird. Dabei kann man nach dem jeweiligen Durchströmen des festen Heizmediums vorsehen, dass Verunreinigungen, Nebenprodukte oder auch das erwünschte Hauptprodukt aus dem Reaktionsmedium entfernt werden. Hierfür sind die bekannten unterschiedlichen Abtrennverfahren geeignet, beispielsweise Absorption auf einer Absorbersubstanz, Separation durch ein Membranverfahren, Ausfällen durch Kühlung oder destillative Abtrennung. Hierdurch kann letztlich eine vollständige Umsetzung des oder der Reaktanten erreicht werden. Dies gilt auch in den Fällen, in denen die chemische Reaktion ohne Abtrennung des Reaktionsprodukts nur bis zu einem Gleichgewichtszustand verläuft.

Die zweckmäßigerweise zu wählende gesamte Kontaktzeit des Reaktionsmediums mit dem induktiv erwärmten Heizmedium hängt von der Kinetik der jeweiligen chemischen Reaktion ab. Die Kontaktzeit ist um so länger zu wählen, je langsamer die erwünschte chemische Reaktion ist. Dies ist im Einzelfall empirisch anzupassen. Als Anhaltspunkt kann gelten, dass vorzugsweise das Reaktionsmedium den Durchflussreaktor mit einer solchen Geschwindigkeit einmal oder mehrmals durchströmt, dass die gesamte Kontaktzeit des Reaktionsmediums mit dem induktiv erwärmten Heizmedium im Bereich von etwa 1 Sekunde bis etwa 2 Stunden liegt, bevor man die Zielverbindung abtrennt. Kürzere Kontaktzeiten sind denkbar, jedoch schwerer zu steuern. Längere Kontaktzeiten können bei besonders trägen chemischen Reaktionen erforderlich sein, verschlechtern jedoch zunehmend die Wirtschaftlichkeit des Verfahrens.

Unabhängig davon, ob man die Reaktion chargenweise oder kontinuierlich in einem Durchflussreaktor durchführt, kann man vorsehen, dass der Reaktor als Druckreaktor ausgelegt ist und die chemische Reaktion bei einem Druck von
oberhalb Atmosphärendruck, vorzugsweise von mindestens 1,5 bar durchgeführt wird. Bekanntermaßen kann hierdurch die Produktausbeute erhöht werden, wenn die Produktbildung (Bildung der Zielverbindung) mit einer Volumenverminderung verbunden ist. Bei zwei oder mehr möglichen Reaktionen kann die Bildung desjenigen Produkts bevorzugt werden, das die stärkste Volumenverminderung zur Folge hat.

Vorzugsweise führt man das erfindungsgemäße Verfahren so durch, dass das Reaktionsmedium im Reaktor unter den eingestellten Reaktionsbedingungen (insbesondere Temperatur und Druck) als Flüssigkeit vorliegt. Hierdurch sind, bezogen auf das Reaktorvolumen, in der Regel bessere Volumen/Zeit-Ausbeuten möglich als bei Reaktionen in der Gasphase.

Es versteht sich von selbst, dass die Natur des Heizmediums und die Auslegung des Induktors so an einander angepasst werden müssen, dass sich die erwünschte Aufheizung des Reaktionsmediums realisieren lässt. Eine kritische Größe hierfür ist einerseits die in Watt ausdrückbare Leistung des Induktors sowie die Frequenz des vom Induktor erzeugten Wechselfelds. Prinzipiell muss die Leistung umso höher gewählt werden, je größer die Masse des induktiv zu erwärmenden Heizmediums ist. In der Praxis ist die erzielbare Leistung insbesondere durch die Möglichkeit begrenzt, den zur Versorgung des Induktors erforderlichen Generator zu kühlen.

Geeignet sind Induktoren, die ein Wechselfeld mit einer Frequenz im Bereich von 1 bis 100 kHz, vorzugsweise von 10 bis 80 kHz und insbesondere im Bereich von etwa 10 bis etwa 30 kHz erzeugen. Solche Induktoren sowie die zugehörigen Generatoren sind kommerziell erhältlich, beispielsweise von der IFF GmbH in Ismaning (Deutschland).

Man führt also die induktive Erwärmung vorzugsweise mit einem Wechselfeld im Mittelfrequenz-Bereich durch. Gegenüber einer Anregung mit höheren Frequenzen, beispielsweise mit solchen im Hochfrequenzbereich (Frequenzen oberhalb von 0,5, insbesondere oberhalb von 1 MHz) hat dies den Vorteil, dass der Energieeintrag in das Heizmedium besser steuerbar ist. Dies gilt insbesondere dann, wenn das Reaktionsmedium unter den Reaktionsbedingungen als Flüssigkeit vorliegt. Daher ist es im Rahmen der vorliegenden Erfindung bevorzugt, dass das Reaktionsmedium als Flüssigkeit vorliegt und dass Induktoren eingesetzt werden, die ein Wechselfeld im vorstehend genannten Mittelfrequenz-Bereich erzeugen. Dies erlaubt eine wirtschaftliche und gut kontrollierbare Reaktionsführung.

In einer speziellen Ausführungsform des erfindungsgemäßen Verfahrens ist das Heizmedium ferromagnetisch und weist eine Curie-Temperatur im Bereich von etwa 40 bis etwa 250°C auf, die man so auswählt, dass sich die Curie-Temperatur um nicht mehr als 20°C, vorzugsweise nicht mehr als 10°C von der ausgewählten Reaktionstemperatur unterscheidet. Dies führt zu einem inhärenten Schutz vor einer unbeabsichtigten Überhitzung. Das Heizmedium lässt sich durch elektromagnetische Induktion nur bis zu seiner Curie-Temperatur aufheizen, während es bei einer darüber liegenden Temperatur nicht weiter durch das elektromagnetische Wechselfeld erwärmt wird. Selbst bei einer Fehlfunktion des Induktors kann auf diese Weise verhindert werden, dass die Temperatur des Reaktionsmediums unbeabsichtigt auf einen Wert deutlich oberhalb der Curie-Temperatur des Heizmediums ansteigt. Fällt die Temperatur des Heizmediums wieder unter seine Curie-Temperatur ab, lässt es sich erneut durch elektromagnetische Induktion erwärmen. Dies führt zu einer Selbstregelung der Temperatur des Heizmediums im Bereich der Curie-Temperatur.

Das erfindungsgemäße Verfahren ist insbesondere zur Durchführung thermisch induzierbarer Reaktionen geeignet. Eine prinzipielle Begrenzung der möglichen Reaktionstypen besteht dabei nicht, sofern nicht Reaktionsbedingungen (wie beispielsweise pH-Wert) gewählt werden oder Edukte verwendet bzw. Produkte gebildet werden, die die das Heizmedium zerstören. Beispielsweise sind chemische Reaktionen durchführbar, bei denen mindestens eine chemische Bindung zwischen 2 C-Atomen oder zwischen einem C-Atom und einem Atom X gebildet, gespalten oder umgelagert wird, wobei X ausgewählt ist aus: H, B, O, N, S, P, Si, Ge, Sn, Pb, As, Sb, Bi und Halogenen. Hierbei kann es sich auch um eine Umlagerung chemischer Bindungen handeln, wie es beispielsweise bei Cycloadditionen und Diels-Alder-Reaktionen geschieht. Beispielsweise kann die thermisch induzierbare Reaktion mindestens einen der folgenden Reaktionstypen entsprechen: Oxidation, Reduktion (einschließlich Hydrierung), Fragmentierung, Addition an eine Doppel- oder Dreifachbindung (incl. Cycloaddition und Diels-Alder-Reaktion), Substitution (SN1 oder SN2, radikalisch), insbesondere aromatische Substitution, Eliminierung, Umlagerung, Kreuzkupplung, Metathesereaktion, Bildung von Heterocyclen, Etherbildung, Esterbildung bzw. Umesterung, Amin-oder Amidbildung, Urethanbildung, pericyclische Reaktion, Michael-Addition, Kondensation, Polymerisation (radikalisch, anionisch, kationisch), Pfropfung von Polymeren. ("Polymer-Grafting").

Für Reduktions- bzw. Hydrierungsreaktionen eignen sich als Reduktionsmittel bzw. Wasserstoffquelle beispielsweise: Cycloalkene wie Cyclohexen, Alkohole wie Ethanol, anorganische Hydrierungsreagenzien wie Natriumborhydrid oder Natriumaluminiumhydrid.

Beispielsweise können Fette oder Öle fragmentiert werden. Dies kann in Lösung, aber auch ohne Lösungsmittel in Substanz erfolgen. In letzterem Fall stellt das Fett bzw. Öl als solches das gesamte Reaktionsmedium dar.

Selbstverständlich sind auch Reaktionen möglich, die zu anorganischen Zielprodukten führen.

Die nachfolgenden Beispiele zeigen chemische Reaktionen, die mit dem erfindungsgemäßen Verfahren in einem Durchflussreaktor im Labormaßstab durchgeführt wurden. Die vorliegende Erfindung ist selbstverständlich hierauf nicht beschränkt.

### Ausführungsbeispiele

Die Erfindung wurde labormäßig erprobt. Als Rohrreaktoren wurden Glasrohre von 10 cm Länge und unterschiedlichen Innen- und Außendurchmesser benutzt. An beiden Enden wurden die Rohre mit Verschraubungen versehen, um HPLC-Anschlüsse und die passenden Schläuche anbringen zu können.

Der Verwendete Induktor hatte folgende Leistungsmerkmale: Induktivität: 134 µHenry, Windungszahl der Spule: = 2·16, Querschnittsfläche = 2.8 mm² (Die Querschnittsfläche ergibt sich aus der Anzahl der verwendeten Leiterdrähte in dem Induktor und ihrem Durchmesser.) Der Durchmesser des Spalts zur Aufnahme der Rohrreaktoren betrug 12 mm. Der Induktor wurde bei allen Versuchen mit einer Frequenz von 25 kHz betrieben.

In den durchgeführten Experimenten wurde die festgelegte Frequenz von 25 kHz konstant gelassen und lediglich über die PWM (PWM = Ein- und Ausschalten eines Rechtecksignals bei fester Grundfrequenz) eine Steuerung der Erwärmung vorgenommen. Im Weiteren wird die PWM in ‰ angegeben. Die Messung der induzierten Temperatur wurde mit einem Thermoelement und einem Infrarotthermometer durchgeführt. Das Thermoelement wurde direkt hinter dem Reaktor im Fluid angebracht, um eine möglichst genaue Messung zu ermöglichen. Dabei musste auf Grund der metallischen Bauteile des Temperaturfühlers jedoch ein Mindestabstand von 4 cm eingehalten werden. Als zweite Temperaturmessung wurde ein Laserinfrarotthermometer mit Scharfpunktoptik verwendet. Der Messpunkt hatte einen Durchmesser von 1 mm. Mit dieser Methode sollte die Oberflächentemperatur des Reaktors gemessen werden, um dadurch einen zweiten Messpunkt für die Temperaturbestimmung zu erhalten. Bei einer Infrarotmessung ist der Emissionsfaktor des Materials eine wichtige Konstante. Er ist ein Maß für die Wärmeabstrahlung. Es wurde mit einem Emissionsfaktor von 0.85 gearbeitet, was einem Durchschnittsglas entspricht.

### Aufheizversuche mit unterschiedlichen Heizmedien:

Die Versuche wurden bei einer Frequenz von 25 kHz mit einer EW 5 Anlage (Leistung 5 Watt) mit trockenen Pulvern (ohne Durchfluss) durchgeführt. Die Erwärmungszeit betrug dabei jeweils 10 Minuten und die Messung der Temperatur wurde mit einem Pyrometer durchgeführt. Folgende Heizmedien wurden getestet:
a) MagSilica^{®} 58/85 der Firma Evonik (früher Degussa),
b) Manganferrit-Pulver der Firma SusTech GmbH, Darmstadt,
c) Bayferrox^{®} 318 M: synthetisches alpha-Fe₃O₄ von Harold Scholz & Co. GmbH,
d) Mangan-Zink-Ferrit, mit Ölsäure oberflächenbeschichtet, Ferritanteil 51,7 Gew.-%, Firma SusTech GmbH, Darmstadt

Dabei wurden nach 10 Minuten folgende Temperaturen erreicht:

| Probe | PWM = 300 ‰ | PWM = 400 ‰ |
|---|---|---|
| a) | 170 °C | 220 °C |
| b) | 130 °C | 150 °C |
| c) | 70 °C | 150 °C |
| d) | 60 °C | 65 °C |

### Durchgeführte Reaktionen mit unterschiedlichen Heizmedien:

Bei den Reaktionen wurden jeweils etwa 3.3 g des genannten Materials in den Reaktor eingefüllt, um die gewünschte Erwärmung durch den Induktor zu erreichen.

### Manganferrit (b):

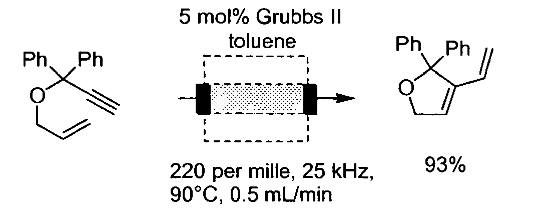

### Bayferrox (c):

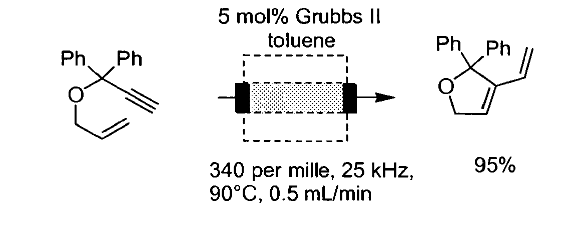

Weiterhin wurde die Erwärmung von gerollter Kupferfolie durch elektromagnetische Induktion überprüft: Die Folie erwärmte sich bei einer Frequenz von 20 kHz und einem PWM von nur 175 ‰ nach weniger als 10 Minuten auf > 160 °C.

Als Heizmedium für die nachstehenden Versuche wurde MagSilica^{®} 58/85 der Firma Evonik (früher Degussa) eingesetzt, das gegebenenfalls gemäß nachstehen Verfahren oberflächlich modifiziert wurde.

### Oberflächenmodifizierung des Heizmediums mit Katalysatoren:

Bei der Herstellung der Katalysatoren wurden Schüttler verwendet, um eine Durchmischung der Substrate zu gewährleisten. Beim Waschen stellten sich herkömmliche Filterpapiere als ungeeignet heraus, da die Poren zu schnell verstopften. Daher wurde der Feststoff bei jedem Waschschritt zentrifugiert. Die Überprüfung der magnetischen Eigenschaften bzw. die magnetische Separation wurde mit einem handelsüblichen Magneten durchgeführt.

### Darstellung Katalysator 7:

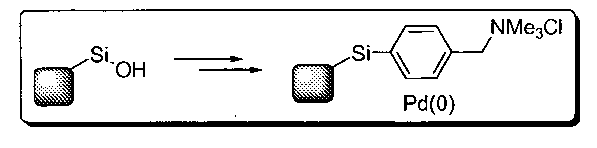

### 1. Stufe: Katalysatorvorstufe 14

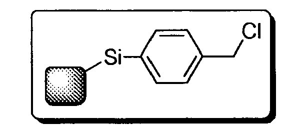

MagSilica 50/85^{®} (15.0 g) wird für 2 h in bidest. H₂O (150 mL) am Rückfluss erhitzt und anschließend im Hochvakuum getrocknet. Der Feststoff wird in Toluol (180 mL) aufgeschlämmt und mit (p-Chlormethyl)phenyltrimethoxysilan (15 mL, 68.1 mmol) für 26 h geschüttelt. Die Reaktionsmischung wird für 3 h unter Rückfluss erhitzt. Nach dem Erkalten wird der Feststoff zentrifugiert und mit Toluol (2x 40 mL) gewaschen. Nach Trocknen im Hochvakuum werden 12.1 g von 14 als schwarzes, magnetisches Pulver erhalten.

### 2. Stufe: Katalysatorvorstufe 61

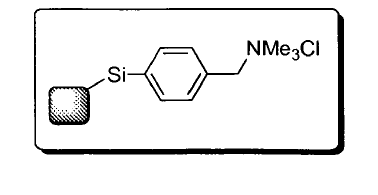

14 (12.0 g) wird in einer mit Trimethylamin gesättigten Lösung von Toluol (350 mL) aufgeschlämmt und für 72 h geschüttelt. Der Feststoff wird zentrifugiert und mit Toluol (3x 40 mL) gewaschen und im Hochvakuum getrocknet. Es werden 12.4 g von 61 als schwarzes, magnetisches Pulver erhalten.

### 3. Stufe: Katalysatorvorstufe 15

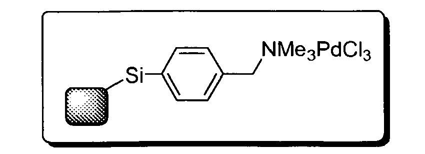

61 (3.0 g) wird in bidest. H₂O (150 mL) aufgeschlämmt und mit einer Lösung von Natriumtetrachloropalladat (100 mg, 0.34 mmol) in bidest. H₂O (10 mL) für 18 h geschüttelt. Der Feststoff wird zentrifugiert und mit bidest. H₂O (2x 40 mL) gewaschen und im Hochvakuum getrocknet. Es werden 2.7 g von 15 als schwarzes, magnetisches Pulver erhalten.

### 4. Stufe: Katalysator 7

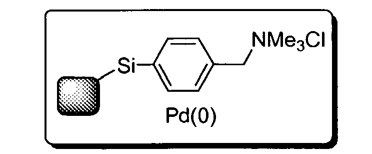

15 (2.7 g) wird in bidest. H₂O (30 mL) aufgeschlämmt und mit einer Lösung von Natriumborhydrid (0.64 g, 16.9 mmol) in bidest. H₂O (15 mL) versehen. Die Reaktionsmischung wird für 5 h geschüttelt, zentrifugiert und mit bidest. H₂O, ges. NaCl-Lösung und H₂O gewaschen (jeweils 40 mL) und im Hochvakuum getrocknet. Es werden 2.6 g von 7 als schwarzes, magnetisches Pulver erhalten. Die Beladung des Katalysators beträgt 6.7 10⁻⁵ mmol Pd/mg Katalysator (ICP-MS Spurenanalyse).

### Darstellung Katalysator 6:

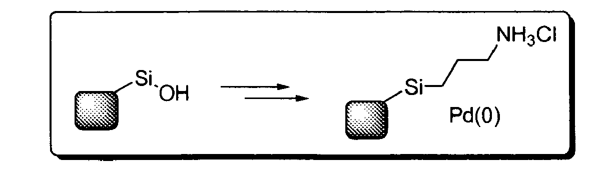

### 1. Stufe: Katalysatorvorstufe 12

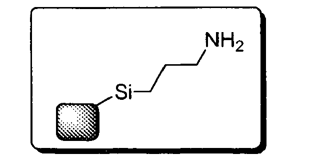

MagSilica 50/85^{®} (6.0 g) wird für 6 h im Hochvakuum bei 200 °C getrocknet und anschließend unter Schutzgasatmosphäre in abs. Toluol (150 mL) aufgeschlämmt. Es wird 3-Aminopropyltrimethoxysilan (4.5 mL, 25.3 mmol) in abs. Toluol (10 mL) hinzugefügt. Die Reaktionsmischung wird für 16 h geschüttelt und zentrifugiert. Der Feststoff wird mit abs. Toluol (2x 40 mL) und wässrigem Toluol (1x 40 mL) gewaschen und anschließend im Hochvakuum getrocknet. Es werden 5.3 g von 12 als schwarzes, magnetisches Pulver erhalten.

### 2. Stufe: Katalysatorvorstufe 62

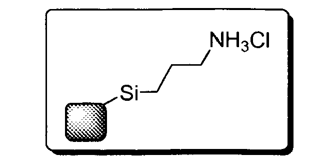

12 (4.0 g) wird unter Schutzgasatmosphäre in abs. Diethylether (180 mL) aufgeschlämmt und mit HBF₄·OEt₂ (10 mL, 38.9 mmol) versehen. Die Reaktionsmischung wird für 2 h geschüttelt. Anschließend wird der Feststoff zentrifugiert und mit Diethylether, wässrigem Diethylether, ges. NaCl-Lösung und bidest. H₂O (jeweils 40 mL) gewaschen und im Hochvakuum getrocknet. Es werden 3.5 g von 62 als schwarzes, magnetisches Pulver erhalten.

### 3. Stufe: Katalysatorvorstufe 13

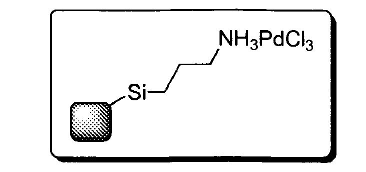

### 62 (3.0 g) wird in bidest. H₂O (150 mL) aufgeschlämmt und mit Natriumtetrachloropalladat (0.5 g, 1.7 mmol) in bidest. H₂O (10 mL) versehen. Die Reaktionsmischung wird für 12 h geschüttelt. Der Feststoff wird mit bidest. H₂O gewaschen bis die Waschlösung nur noch schwach gelblich ist (3x 40 mL) und anschließend im Hochvakuum getrocknet. Es werden 2.8 g von 13 als schwarzes, magnetisches Pulver erhalten. Die Beladung des Katalysators beträgt 3.7 10⁻⁵ mmol Pd/mg Katalysator (ICP-MS Spurenanalyse).

### 4. Stufe: Katalysator 6

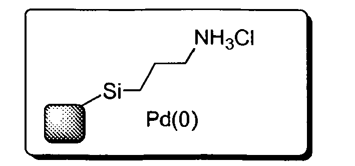

13 (2.5 g) wird in bidest. H₂O (40 mL) aufgeschlämmt und mit einer Lösung von Natriumborhydrid (0.64 g, 16.9 mmol) in bidest. H₂O (15 mL) versehen. Die Reaktionsmischung wird geschüttelt bis die Gasentwicklung aufhört. Der Feststoff wird mit bidest. H₂O, gesättigter NaCl-Lösung und bidest. H₂O gewaschen (jeweils 40 mL) und im Hochvakuum getrocknet. Es werden 2.3 g von 6 als schwarzes, magnetisches Pulver erhalten. Die Beladung des Katalysators beträgt 3.7 10⁻⁵ mmol Pd/mg Katalysator.

### Katalysator 8

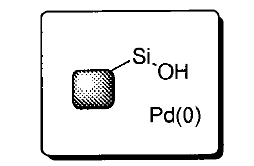

MagSilica 50/85^{®} (2.0 g) wird in EtOH aufgeschlämmt und mit einer Lösung von Palladiumnitratdihydrat (84 mg, 0.32 mmol) in EtOH (10 mL) bei 50 °C für 30 min gerührt. Die Reaktionsmischung wird im Vakuum bis zur Trockne eingeengt. Nach Trocknen im Vakuum werden 1.92 g von 8 als schwarzes, magnetisches Pulver erhalten. Die Beladung des Katalysators beträgt 11.3 . 10⁻⁵ mmol Pd/mg Katalysator.

### Darstellung Katalysator 9:

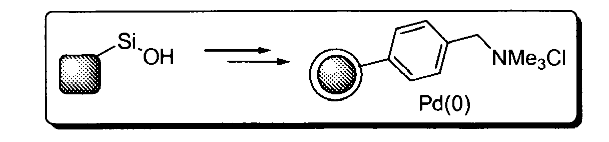

### 1. Stufe: Katalysatorvorstufe 63

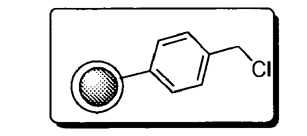

MagSilica 50/85^{®} (3.0 g) wird für 2 h in bidest. H₂O (50 mL) unter Rückfluss erhitzt und im Hochvakuum getrocknet. Der schwarze Feststoff wird in Dodecan (18 mL) mit DVB (0.41g, 5.3 m%), VBC (7.11 g, 94.7 m%) und AIBN (37.5 mg, 0.5 m%) versehen und mit einem KPG-Rührer bei 70 °C für 16 h gerührt. Die erhaltene schwarze Suspension wird in einem Soxlett-Extraktor für 13 h mit Chloroform gereinigt. Das Produkt wird mit einem Magneten von dem übrigen Polymer getrennt und im Hochvakuum getrocknet. Es werden 4.4 g von 63 als schwarz-grünliches, magnetisches Pulver erhalten.

### 2. Stufe: Katalysatorvorstufe 64

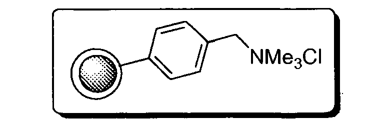

63 (4.4 g) wird in einer mit Trimethylamin gesättigten Lösung von Toluol (350 mL) aufgeschlämmt und für 93 h geschüttelt. Der Feststoff wird zentrifugiert und mit Toluol (3x 40 mL) gewaschen. Nach Trocknen im Hochvakuum wird ein schwarz-grünliches, magnetisches Pulver erhalten. Es werden 4.1 g von 63 erhalten.

### 3. Stufe: Katalysatorvorstufe 65

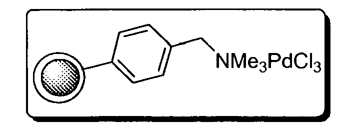

64 (4.1 g) wird in bidest. H₂O (150 mL) aufgeschlämmt und mit einer Lösung von Natriumtetrachloropalladat (700 mg, 2.38 mmol) in bidest. H₂O (10 mL) für 17 h geschüttelt. Der Feststoff wird zentrifugiert, mit bidest. H₂O (3x 40 mL) gewaschen und im Hochvakuum getrocknet. Es wird ein schwarz-grünlicher, magnetischer Feststoff erhalten. Das Rohprodukt wird ohne Trocknung in der folgenden Reaktion eingesetzt.

### 4. Stufe: Katalysator 9

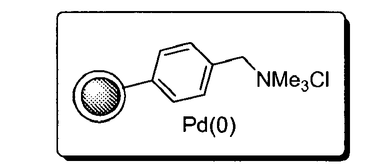

65 (4.0 g) wird in bidest. H₂O (50 mL) aufgeschlämmt und mit einer Lösung von Natriumborhydrid (0.5 g, 13.2 mmol) in bidest. H₂O (15 mL) versehen. Die Reaktionsmischung wird für 2 h geschüttelt, zentrifugiert, mit bidest. H₂O, gesättigter NaCl-Lösung und H₂O gewaschen (jeweils 40 mL) und im Hochvakuum getrocknet. Es werden 3.3 g von 9 als schwarz-grünliches, magnetisches Pulver erhalten.

Nachfolgend werden einige chemische Reaktionen angegeben, die mit dem erfindungsgemäßen Verfahren durchgeführt wurden. In allen Fällen wurde Dimethylformamid (DMF) als Lösungsmittel verwendet. Zum Vergleich wurden dieselben Reaktionen auch mit konventioneller Erwärmung in einem Heizbad mit denselben Temperaturen und Reaktionszeiten durchgeführt. In den Tabellen werden die Reaktionsausbeuten im Vergleich angegeben, wobei "thermisch" der Umsatz im Heizbad (Vergleich) und "induktiv" der Umsatz nach dem erfindungsgemäßen Verfahren bedeuten. Bei dem erfindungsgemäßen Verfahren wurde die Gesamt-Reaktionszeit dadurch erreicht, dass das Reaktionsmedium im Kreis geführt wurde und den Reaktor entsprechend oft durchströmte.

Um bei dem erfindungsgemäßen Verfahren angenähert dieselbe Reaktionstemperatur wie bei dem thermischen Verfahren zu erreichen, wurden Vorversuche zum Aufheizverhalten durchgeführt. Hierzu wurde der Rohrreaktor mit einer Mischung aus MagSilica^{®} und Sand (ca. 67 Vol-% MagSilica und 33 Vol-% Sand) und mit DMF durchströmt. Der Induktor wurde stets mit 25 kHz betrieben. Diese Bedingungen wurden auch bei den einzelnen Reaktionen eingehalten.

### Ergebnis der Heizversuche:

**Tabelle 1: Heiztabelle. Messung jeweils bis zum Konstantbleiben der Temperatur mit 25 kHz, 2 mL/min in DMF**

| PWM [‰] | Messzeit [min] | Außentemp. [°C]^{a} | Fluidtemp. [°C]^{b} |
|---|---|---|---|
| 600 | 2 | > 170 | -^{c} |
| 400 | 4 | > 170 | -^{c} |
| 350 | 10 | > 170 | 72 |
| 325 | 15 | 145 | 60 |
| 300 | 15 | 136 | 49 |
| 250 | 15 | 71 | 37 |
| 225 | 15 | 54 | 20 |
| 200 | 15 | 33 | 19 |

| | | | |
|---|---|---|---|
| , ^{a} Messung über Temperaturfühler, Messung über Infrarotthermometer, keine Messung wegen Fluidverdampfung möglich. | | | |

### Reaktionsbeispiele:

| Tabelle 2: Heck-Mizoroki-Kupplungen, 1 mmol Arylhalogenid, 3 Äq. Styrol, 3 Äq. *n*-Bu₃N, 2.8 mol% Katalysator 7, Reaktionszeit jeweils 1 h, Flussrate 2 mL/min, PWM = 325 ‰. | | | | | |
|---|---|---|---|---|---|
| Reaktionsprinzip: | | | | | |
| 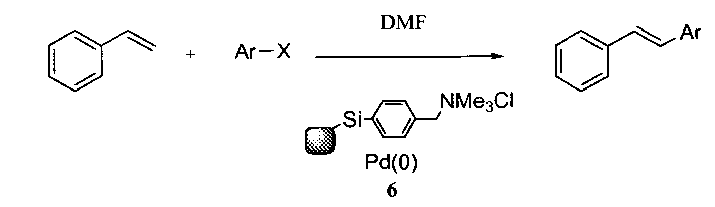 | | | | | |
| Beisp. Nr. | Arylhalogenid | Alken | Produkt | Umsatz thermisch [%] | Umsatz induktiv [%]^{a} |
| 1 | 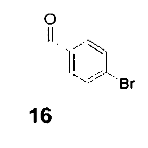 | 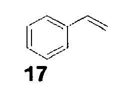 | 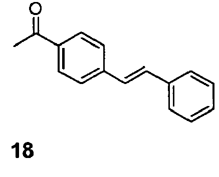 | 0 | 7.5 |
| 2 | 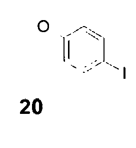 | 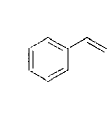 | 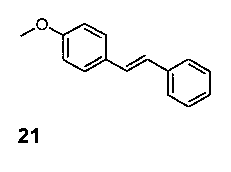 | 60.0 | 84.2 |

**Tabelle 3: Weitere Katalysatoren; Suzuki-Miyaura-Reaktion**

| 2 mL/min, Reaktionszeit jeweils 1h,: 0.5 mmol Arylhalogenid, 1.5 Äq. Boronsäure, 2.4 Äq. CsF, PWM = 750 ‰ | | | |
|---|---|---|---|
| ^{a} 1 mol%, ^{b} 2.8 mol%,. | | | |
| Suzuki-Miyaura-Reaktion: | | | |
| 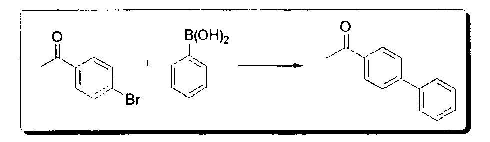 | | | |
| Katalysator | Beisp. Nr. | Umsatz Thermisch [%] | Umsatz induktiv [%] |
| Najera-Katalysator ^{a} | | | |
| 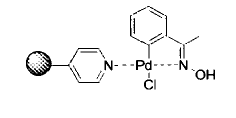 | 3 | 23.0 | 49.1 |
| 5 % Pd/Aktivkohle ^{a} | 4 | 5.8 | 58.8 |
| Raschig-Ring-Katalysator ^{a} 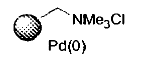 | 5 | 50.2 | 78.7 |
| Katalysator 8 ^{b} 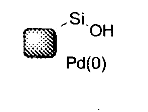 | 6 | - | 97.4 |

### Beispiel 7:

Umesterung von Zimtsäureethylester 59 zu Zimtsäuremethylester 60. Dieser Versuch wurde in Anlehnung an eine Literaturvorschrift durchgeführt (K. Jansson, T. Fristedt, A. Olsson, B. Svensson, S. Jönsson, J. Org. Chem., 2006, 71, 1658-1667). Dabei wurde ein Überschuss an Natriummethanolat verwendet, um das Gleichgewicht der Umesterung zu dem gewünschten Produkt zu verschieben. Mit induktiver Heizung (Flussrate: 2 mL/min, PWM = 240 ‰) konnte nach 25 min 93 % Produkt isoliert werden.

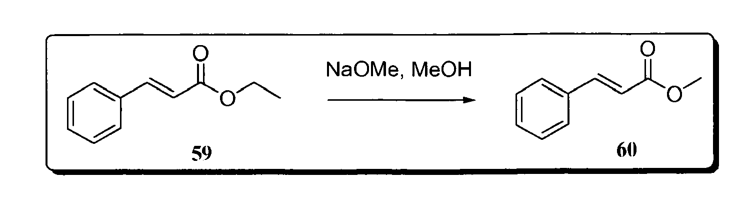

Weitere thermische Reaktionen im Durchfluss mit induktiver Beheizung
(Dabei bedeut: prom = PWM in ‰. Die Zahlen in % unter der Produktformel geben die Produktausbeute an.)

### Beispiel 8: Heterocyclen-Synthese:

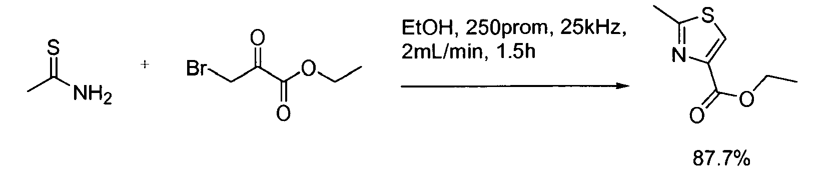

### Beispiel 9: Hartwig-Buchwald-Kupplung:

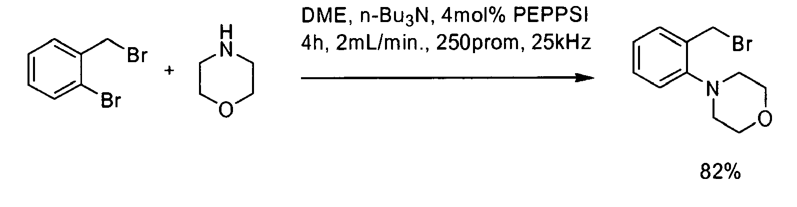

### Beispiel 10: Claissen-Umlagerung:

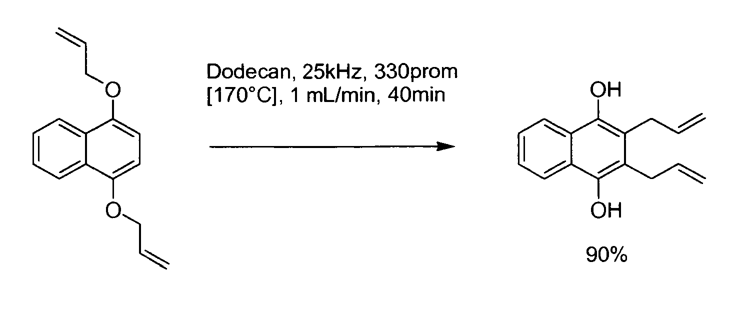

### Beispiel 11: Decarboxylierung

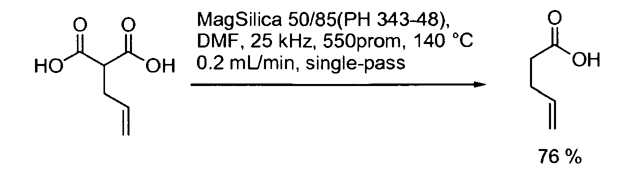

(PH 343-48 gibt die Chargennummer des MagSilica^{R} an.)

### Beispiel 12: Hydrierung

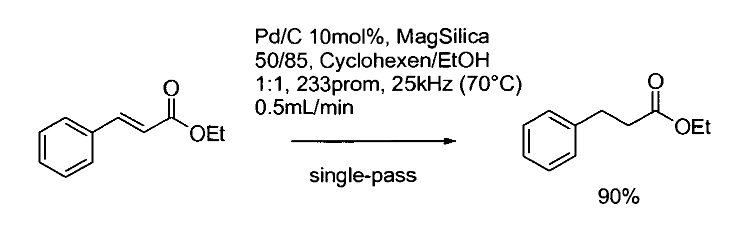

### Beispiel 13: Reduktion

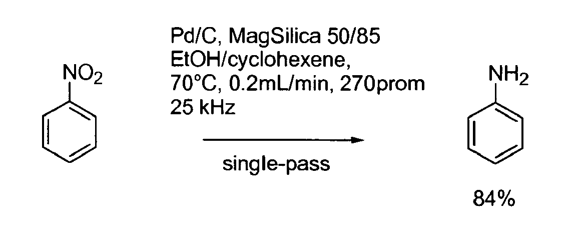

Bei den Beispielen 12 und 13 wurde der Katalysator Pd/C (Palladium auf Aktivkohle) in Abmischung mit MagSilica^{R} eingesetzt. Als Reduktionsmittel (Wasserstoffquelle) dient im wesentlichen das Cyclohexen.

### Beispiel 14: Umlagerung unter C - C - Verknüpfung

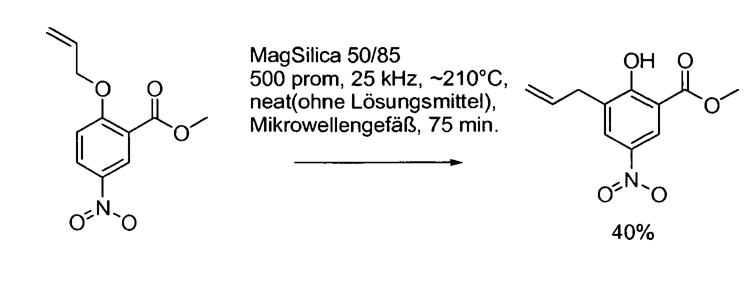

## Patentansprüche

1. Verfahren zum Durchführen einer chemischen Reaktion zur Herstellung einer Zielverbindung durch Erwärmen eines Reaktionsmediums enthaltend mindestens einen ersten Reaktanten in einem Reaktor, wodurch eine chemische Bindung innerhalb des ersten Reaktanten oder zwischen dem ersten und einem zweiten Reaktanten gebildet oder verändert wird, wobei man das Reaktionsmedium in Kontakt mit einem durch elektromagnetische Induktion erwärmbaren festen Heizmedium bringt, das sich innerhalb des Reaktors befindet und das von dem Reaktionsmedium umgeben ist, und das Heizmedium durch elektromagnetische Induktion mit Hilfe eines Induktors erwärmt, wobei sich aus dem ersten Reaktanten oder aus dem ersten und einem zweiten Reaktanten die Zielverbindung bildet und wobei man die Zielverbindung von dem Heizmedium abtrennt, **dadurch gekennzeichnet, dass** das Reaktionsmedium im Reaktor als Flüssigkeit vorliegt und dass der Induktor ein Wechselfeld mit einer Frequenz im Bereich von 1 bis 100 kHz erzeugt.

2. Verfahren nach Anspruch 1, **dadurch gekennzeichnet, dass** das Heizmedium ausgewählt ist aus Partikeln elektrisch leitfähiger und/oder magnetisierbarer Festkörper, wobei die Partikel eine mittlere Teilchengröße im Bereich von 1 bis 1000 nm haben.

3. Verfahren nach Anspruch 2 , **dadurch gekennzeichnet, dass** das Heizmedium ausgewählt ist aus Partikeln magnetisierbarer Festkörper, wobei jedes Partikel mindestens einen Kern aus einem magnetisierbarem Material enthält, der von einem nicht magnetischen Material umhüllt ist.

4. Verfahren nach einem der Ansprüche 2 und 3, **dadurch gekennzeichnet, dass** das Heizmedium ausgewählt ist aus Partikel magnetisierbaren Festkörper und dass diese in Mischung mit weiteren Partikeln vorliegen, die nicht durch elektromagnetische Induktion erwärmbar sind.

5. Verfahren nach einem oder mehreren der Ansprüche 1 bis 4, **dadurch gekennzeichnet, dass** das feste Heizmedium oberflächlich mit einer für die chemische Reaktion katalytisch wirkenden Substanz belegt ist.

6. Verfahren nach einem oder mehreren der Ansprüche 1 bis 5, **dadurch gekennzeichnet, dass** man die chemische Reaktion chargenweise durchführt, wobei man während der Reaktion das Reaktionsmedium und das feste Heizmedium relativ zueinander bewegt.

7. Verfahren nach Anspruch 6, **dadurch gekennzeichnet, dass** sich das Reaktionsmedium zusammen mit Partikeln des Heizmediums in einem Reaktionsbehälter befindet und mit Hilfe eines im Reaktionsmedium befindlichen Bewegungselements.bewegt wird, wobei das Bewegungselement als Induktor eingerichtet ist, durch den die Partikel des Heizmediums erwärmt werden.

8. Verfahren nach einem oder mehreren der Ansprüche 1 bis 5, **dadurch gekennzeichnet, dass** man die chemische Reaktion in einem Durchflussreaktor durchführt, der zumindest teilweise mit dem festen Heizmedium gefüllt ist und hierdurch mindestens eine durch elektromagnetische Induktion erwärmbare Heizzone aufweist, wobei das Reaktionsmedium den Durchflussreaktor durchströmt und wobei sich der Induktor außerhalb des Reaktors befindet.

9. Verfahren nach Anspruch 8, **dadurch gekennzeichnet, dass** das Reaktionsmedium den Durchflussreaktor mit einer solchen Geschwindigkeit einmal oder mehrmals durchströmt, dass die gesamte Kontaktzeit des Reaktionsmediums mit dem Heizmedium im Bereich von einer Sekunde bis 2 Stunden liegt.

10. Verfahren nach einem oder mehreren der Ansprüche 1 bis 9, **dadurch gekennzeichnet, dass** der Reaktor als Druckreaktor ausgelegt ist und die chemische Reaktion bei einem Druck von oberhalb Atmosphärendruck, vorzugsweise von mindestens 1,5 bar durchgeführt wird.

11. Verfahren nach einem oder mehreren der Ansprüche 1 bis 10, **dadurch gekennzeichnet, dass** das Heizmedium ferromagnetisch ist und eine Curie-Temperatur im Bereich von 40 bis 250°C aufweist und so ausgewählt wird, dass sich die Curie-Temperatur um nicht mehr als 20°C, von der ausgewählten Reaktionstemperatur unterscheidet.

12. Verfahren nach einem oder mehreren der Ansprüche 1 bis 11, **dadurch gekennzeichnet, dass** bei der chemischen Reaktion eine chemische Bindung zwischen zwei C-Atomen oder zwischen einem C-Atom und einem Atom X gebildet wird, wobei X ausgewählt ist aus: H, B, O, N, S, P, Si, Ge, Sn, Pb, As, Sb, Bi und Halogen.

## Claims

1. A process for carrying out a chemical reaction for producing a target compound by heating a reaction medium comprising at least one first reactant in a reactor, whereby a chemical bond within the first reactant or between the first and a second reactant is formed or modified, wherein the reaction medium is brought into contact with a solid heating medium that can be heated by electromagnetic induction and that is inside the reactor and is surrounded by the reaction medium, and said heating medium is heated by electromagnetic induction with the aid of an inductor, wherein the target compound is formed from the first reactant or from the first and a second reactant and wherein said target compound is separated from the heating medium, **characterised in that** the reaction medium in the reactor is present as a liquid and the inductor generates an alternating field with a frequency in the range of 1 to 100 kHz.

2. The process according to claim 1, **characterised in that** the heating medium is selected from particles of electrically conductive and/or magnetisable solids, wherein the mean particle size of the particles is between 1 and 1000 nm.

3. The process according to claim 2, **characterised in that** the heating medium is selected from particles of magnetisable solids, wherein each particle comprises at least one core of a magnetisable material which is encapsulated by a non-magnetic material.

4. The process according to one of claims 2 and 3, **characterised in that** the heating medium is selected from particles of magnetisable solids, and that these are present in a mixture with additional particles that are not able to be heated by electromagnetic induction.

5. The process according to one or more of claims 1 to 4, **characterised in that** the solid heating medium is surface coated with a substance that is catalytically active for the chemical reaction.

6. The process according to one or more of claims 1 to 5, **characterised in that** the chemical reaction is carried out batch-wise, wherein the reaction medium and the solid heating medium move relative to each other during the reaction.

7. The process according to claim 6, **characterised in that** the reaction medium is present together with particles of the heating medium in a reaction vessel, and is moved with the help of a moving element located in the reaction medium, wherein the moving element is arranged as an inductor, by which the particles of the heating medium are heated.

8. The process according to one or more of claims 1 to 5, **characterised in that** the chemical reaction is carried out in a flow-through reactor that is at least partially filled with the solid heating medium and thereby possesses at least one heating zone that can be heated by electromagnetic induction, wherein the reaction medium flows through the flow-through reactor and wherein the inductor is located outside the reactor.

9. The process according to claim 8, **characterised in that** the reaction medium flows once or a plurality of times through the flow-through reactor with a speed such that the total contact time of the reaction medium with the heating medium is in the range of one second to 2 hours.

10. The process according to one or more of claims 1 to 9, **characterised in that** the reactor is configured as a pressure reactor and the chemical reaction is carried out at a pressure greater than atmospheric pressure, preferably under at least 1.5 bar.

11. The process according to one or more of claims 1 to 10, **characterised in that** the heating medium is ferromagnetic and exhibits a Curie temperature in the range of 40 to 250 °C, and is selected such that the Curie Temperature does not differ by more than 20 °C from the selected reaction temperature.

12. The process according to one or more of claims 1 to 11, **characterised in that** a chemical bond is formed between two carbon atoms or between a carbon atom and an atom X in the chemical reaction, wherein X is selected from: H, B, O, N, S, P, Si, Ge, Sn, Pb, As, Sb, Bi and halogen.

## Revendications

1. Procédé pour la mise en oeuvre d'une réaction chimique afin d'obtenir un composé cible par réchauffement d'un milieu réactionnel contenant au moins un premier réactif dans un réacteur, si bien que l'on obtient la formation ou la modification d'une liaison chimique au sein du premier réactif ou bien entre le premier réactif et un deuxième réactif, procédé dans lequel on amène le milieu réactionnel en contact avec un milieu chauffant solide qui peut être réchauffé par induction électromagnétique, le milieu en question se trouvant au sein du réacteur et étant entouré par le milieu réactionnel, et on réchauffe le milieu chauffant par induction électromagnétique à l'aide d'un inducteur ; dans lequel on obtient le composé cible à partir du premier réactif ou bien à partir du premier réactif et d'un deuxième réactif ; et dans lequel on sépare le composé cible du milieu chauffant, **caractérisé en ce que** le milieu réactionnel est présent dans le réacteur sous la forme d'un liquide et **en ce que** l'inducteur génère un champ alternatif avec une fréquence dans la plage de 1 à 100 kHz.

2. Procédé selon la revendication 1, **caractérisé en ce que** le milieu chauffant est choisi parmi des particules de corps solides électroconducteurs et/ou magnétisables, les particules possédant une granulométrie moyenne dans la plage de 1 à 1000 nm.

3. Procédé selon la revendication 2, **caractérisé en ce que** le milieu chauffant est choisi parmi les particules de corps solides magnétisables, chaque particule contenant au moins un noyau constitué d'un matériau magnétisable qui est entouré d'un matériau antimagnétique.

4. Procédé selon l'une quelconque des revendications 2 et 3, **caractérisé en ce que** le milieu chauffant est choisi parmi des particules de corps solides magnétisables et **en ce que** lesdites particules sont présentes en mélange avec d'autres particules qui ne peuvent pas être réchauffées par induction non-magnétique.

5. Procédé selon une ou plusieurs des revendications 1 à 4, **caractérisé en ce que** le milieu chauffant solide est recouvert à sa surface d'une substance ayant un effet catalytique pour la réaction chimique.

6. Procédé selon une ou plusieurs des revendications 1 à 5, **caractérisé en ce qu'**on met en oeuvre la réaction chimique en discontinu, en déplaçant le milieu réactionnel et le milieu chauffant solide l'un par rapport à l'autre au cours de la réaction.

7. Procédé selon la revendication 6, **caractérisé en ce que** le milieu réactionnel se trouve dans un récipient de réaction de manière conjointe avec des particules du milieu chauffant et est mis en mouvement à l'aide d'un élément mobile se trouvant dans le milieu réactionnel, l'élément mobile étant réalisé sous la forme d'un inducteur par lequel les particules du milieu chauffant sont réchauffées.

8. Procédé selon une ou plusieurs des revendications 1 à 5, **caractérisé en ce qu'**on met en oeuvre la réaction chimique dans un réacteur à écoulement continu qui est rempli au moins en partie avec le milieu chauffant solide et qui présente de cette manière au moins une zone de chauffage qui peut être réchauffée par induction électromagnétique, le milieu réactionnel traversant le réacteur à écoulement continu et l'inducteur étant situé à l'extérieur du réacteur.

9. Procédé selon la revendication 8, **caractérisé en ce que** le milieu réactionnel traverse à une ou plusieurs reprises le réacteur à écoulement continu avec une vitesse telle que le temps de contact total du milieu réactionnel avec le milieu chauffant se situe dans la plage de 1 seconde à 2 heures.

10. Procédé selon une ou plusieurs des revendications 1 à 9, **caractérisé en ce que** le réacteur est conçu sous la forme d'un réacteur sous pression et la réaction chimique est mise en oeuvre sous une pression supérieure à la pression atmosphérique, de préférence sous une pression d'au moins 1,5 bar.

11. Procédé selon une ou plusieurs des revendications 1 à 10, **caractérisé en ce que** le milieu chauffant est de type ferromagnétique et présente une température de Curie dans la plage de 40 à 250 °C, et est choisi de telle sorte que la température de Curie ne diffère pas de plus de 20 °C de la température réactionnelle sélectionnée,.

12. Procédé selon une ou plusieurs des revendications 1 à 11, **caractérisé en ce que**, en ce qui concerne la réaction chimique, il s'agit d'une réaction chimique entre deux atomes de carbone ou bien entre un atome de carbone et un atome X, X étant choisi parmi : H, B, O, N, S, P, Si, Ge, Sn, Pb, As, Sb, Bi et un atome d'halogène.
